# FASCICULE DE BREVET EUROPEEN

(11) **EP 2 988 709 B1**
(45) Date de publication et mention de la délivrance du brevet: **22.03.2017**
(21) Numéro de dépôt: 14726700.9
(22) Date de dépôt: 25.04.2014
(51) Int. Cl.: A61F 5/56

(54) **DISPOSITIF ANTI-RONFLEMENT**
ANTISCHNARCHVORRICHTUNG
ANTI SNORING DEVICE

(30) Priorité: 27.04.2013 FR 1353892
(43) Date de publication de la demande: 02.03.2016
(73) Titulaire: SIMP Société Industrielle de Matériel Paramédical, 69230 Saint Genis Laval (FR); NCT Consultants, 69380 Lissieu (FR)
(72) Inventeur: FRENAY, Patrick, Michel, Eric, 69340 Francheville (FR); COËT-TOMATIS, Nicolas, Bernard, 69380 Lissieu (FR)
(74) Mandataire: Weber, Jean-François
(86) Numéro de dépôt international: PCT/FR2014/051008
(87) Numéro de publication internationale: WO 2014/174226

(56) Documents cités:
- DE-A1- 2 249 975
- FR-A1- 2 965 714
- US-A- 5 357 981
- US-A1- 2012 167 895

## Description

### DOMAINE TECHNIQUE

La présente invention concerne le domaine technique des dispositifs anti-ronflement susceptibles d'être portés par un utilisateur à la manière d'un vêtement.

### TECHNIQUE ANTERIEURE

Dans le domaine ci-dessus, il a, par exemple, été proposé d'équiper le dos d'une veste de pyjama d'une poche destinée à recevoir une balle de tennis. Ainsi, le porteur de cette veste de pyjama avec la balle de tennis dans la poche dorsale se trouve incommodé lorsqu'il est en position allongé sur le dos de sorte que, pendant son sommeil, il se place naturellement et inconsciemment dans une position couchée sur le côté ou sur le ventre et ne reste jamais en position sur le dos. Or, dans la plupart des cas les ronflements n'apparaissent que dans la position allongée sur le dos qui induit, notamment, un recul de la langue dans la cavité buccale ainsi qu'un relâchement des muscles de la gorge.

Ainsi, une telle veste de pyjama permet de lutter efficacement contre les ronflements. Toutefois, la mise en oeuvre d'une balle de tennis rend le port de la veste inconfortable y compris dans des positions autres que couché sur le dos. De plus, la dureté de la balle de tennis rend douloureux le passage par la position couchée sur le dos de sorte que le porteur de la veste ne peux plus passer pendant son sommeil d'une position couchée sur un côté à une position couchée sur l'autre côté.

En outre, la balle de tennis, bien que reçue dans une poche, n'en reste pas moins relativement mobile, ce qui peut être source d'inconfort et/ou de perte d'efficacité. Il ne peut enfin pas être totalement exclu que la balle puisse s'échapper hors de la poche du pyjama.

Il est donc apparu le besoin d'un autre dispositif anti-ronflement qui, tout en mettant en oeuvre le même principe général que la veste selon l'art antérieur (*i.e.* induire une posture de sommeil non dorsale), offre un meilleur confort de port ainsi qu'une plus grande robustesse et une fiabilité améliorée, tout en étant particulièrement facile, rapide et bon marché à fabriquer.

On connaît par ailleurs du document US-2012/167895 un dispositif qui est pourvu d'un aileron massif (cf. figure 5) et correspond au préambule de la revendication 1.

### EXPOSE DE L'INVENTION

Afin d'atteindre cet objectif, l'invention concerne un dispositif anti-ronflement comprenant un élément en volume ainsi qu'un élément de maintien dans le dos d'un utilisateur dudit élément en volume, ledit élément en volume comprenant au moins un rebord, caractérisé en ce que ledit élément en volume comprend au moins une coque élastiquement déformable et présentant une face proximale sensiblement plane ou concave orientée vers l'élément de maintien, ledit rebord bordant ladite face proximale et se présentant sous la forme d'un volet s'étendant entre une extrémité intérieure attachée à ladite coque et une extrémité extérieure libre, ledit dispositif comprenant par ailleurs un moyen de fixation assurant un assemblage permanent du volet et de l'élément de maintien.

La mise en oeuvre d'un élément en volume qui présente une face proximale plane ou convexe offre un meilleur confort de port à l'utilisateur que la balle de tennis selon l'art antérieur, dans la mesure où la forme plane ou convexe permet, à la partie de la coque orientée vers le dos de l'utilisateur, d'en épouser plus ou moins la forme. De plus, la présence du rebord de fixation immobilise parfaitement la coque sur l'élément de maintien de sorte que la face proximale reste bien orientée vers le dos de l'utilisateur lors du port du dispositif selon l'invention. De plus, la forme de volet dudit rebord permet de faciliter l'assemblage du volet de l'élément de maintien, et de favoriser la stabilité, la robustesse et la durabilité dudit assemblage. En d'autres termes, le rebord se présente sous la forme d'une lèvre qui fait saillie latéralement et qui présente de préférence une certaine souplesse, pour accommoder notamment les mouvements et déformations de l'élément de maintien, lequel présente avantageusement un caractère élastique. De préférence, le volet s'étend sur toute la périphérie de la face proximale, de sorte qu'il affecte dans ce cas une forme sensiblement annulaire, de préférence circulaire, du genre collerette ou jupe.

Grâce à la mise en oeuvre d'un assemblage permanent, l'élément en volume est parfaitement et durablement maintenu en position fonctionnelle, et ne risque pas de se désolidariser intempestivement de l'élément de maintien sous l'effet des mouvements opérés consciemment ou inconsciemment par l'utilisateur au cours de son sommeil. En cela, les moyens de fixation selon l'invention diffèrent totalement de moyens de fixation réversibles, du genre Velcro ® par exemple, ces derniers permettant uniquement une fixation amovible, alors que l'invention vise au contraire une fixation définitive et irréversible. De préférence, le moyen de fixation comprend au moins un point de couture, et de préférence une suite de points de couture, continue ou discontinue, de sorte que le volet est cousu à l'élément de maintien. De façon avantageuse, la fixation de l'élément en volume à l'élément de maintien est exclusivement réalisé par assemblage permanent du volet et de l'élément de maintien, de préférence par couture, ce qui permet d'obtenir de façon très simple et peu onéreuse un ensemble fonctionnel robuste, durable et sûr sur le plan sanitaire (absence de colle).

Le recours à une coque élastiquement déformable permet en outre d'obtenir un bon compromis entre efficacité et confort. Par « *élastiquement déformable* », il convient d'entendre que la coque est susceptible de connaître une déformation de préférence légère lorsque le porteur du dispositif se place en position couché sur le dos par opposition à ce qui se passerait si la coque était réalisée en un matériau dur tel que du bois. A cet égard, la coque est, de préférence, adaptée pour se déformer sous une charge supérieure ou égale à 200 N. Ce caractère élastiquement déformable de la coque, qui lui permet de se déformer très légèrement lorsque l'utilisateur passe en position couché sur le dos, évite que la coque ne blesse l'utilisateur lorsqu'il est sur le dos tout en induisant une gêne suffisante pour que l'utilisateur ne reste pas dans cette position couché sur le dos.

Selon une caractéristique préférentielle de l'invention, la coque de l'élément en volume présente, à l'opposé de la face proximale, une face distale qui possède une forme convexe. Selon une variante de cette caractéristique, la face distale de la coque possède sensiblement une forme de calotte sphérique. Une telle forme permet, de manière avantageuse, de limiter la déformation de la coque lorsqu'elle est élastiquement déformable de sorte que même déformée elle continue d'induire une gêne.

De préférence, la coque possède sensiblement une forme de bulbe, c'est-à-dire qu'elle affecte une forme de protubérance arrondie, avantageusement une forme de sphère ou pseudo-sphère tronquée pour former la face proximale. Une telle forme procure un excellent compromis entre efficacité d'une part et confort d'utilisation d'autre part.

Selon une autre caractéristique préférentielle de l'invention, la coque délimite une cavité qui s'ouvre sur l'extérieur par l'intermédiaire d'une ouverture ménagée à travers ladite face proximale. En d'autres termes, la coque forme avantageusement une enveloppe qui entoure un volume intérieur accessible de l'extérieur uniquement par une ouverture disposée au niveau de la face proximale. En l'espèce, ladite ouverture est obturée par l'élément de maintien, contre lequel vient en appui la face proximale. La face proximale de la coque est ainsi ouverte vers l'élément de maintien, lequel est de préférence poreux, perméable à l'air. Dans ce mode de réalisation, la face proximale présente donc sensiblement une forme annulaire, qui entoure l'ouverture d'accès au volume interne délimité par la coque. Cette ouverture fait office d'évent ce qui facilite la déformation de la coque en permettant un échappement de l'air. A cette fin, la cavité délimitée par la coque est de préférence majoritairement vide, et de façon encore plus préférentielle totalement vide (c'est-à-dire qu'elle est remplie d'air). Il est cependant parfaitement envisageable, sans pour autant que l'on sorte du cadre de l'invention, que ladite cavité soit remplie partiellement ou totalement d'un matériau souple et déformable, du genre mousse par exemple, sans pour autant que l'on sorte du cadre de l'invention. Grâce à la mise en oeuvre d'une face proximale annulaire, les éventuels efforts transmis sur le dos de l'utilisateur par l'élément en volume s'exercent sur une surface réduite en minimisant les efforts exercés directement sur la colonne vertébrale de l'utilisateur.

Selon encore une autre caractéristique de l'invention, la coque possède une hauteur, mesurée entre l'élément de maintien et le sommet de la coque (*i.e.* le sommet de sa face distale), comprise entre 30 mm et 100 mm, et de préférence supérieure à 50 mm.

Selon l'invention, la coque peut être réalisée en tout matériau approprié et, de préférence, en élastomère naturel ou synthétique. Afin d'éviter les risques d'allergie, la coque peut par exemple être réalisée en silicone de grade médical ou alimentaire. De préférence, le volet est réalisé lui aussi en un matériau élastomère. De façon particulièrement avantageuse, le volet vient de matière avec la coque, de sorte qu'il forme avec cette dernière une pièce d'un seul tenant, c'est-à-dire un sous-ensemble unitaire, robuste et compact, conçu pour être attaché à l'élément de maintien.

Selon l'invention l'élément de maintien peut être réalisé de toutes manières appropriées. Dans une forme préférée mais non exclusive de réalisation de l'invention, l'élément de maintien est constitué par une ceinture pectorale qui comprend un corps souple allongé élastiquement déformable dans le sens de la longueur et pourvu, au niveau d'une extrémité au moins, de moyens de fermeture de la ceinture en boucle autour du torse de l'utilisateur. La mise en oeuvre d'une telle ceinture pectorale, qui se présente avantageusement sous la forme d'une bande de tissu élastique, permet d'offrir un très bon confort de port sous un vêtement de nuit. De plus, cette ceinture peut couvrante ne tient pas trop chaud de sorte que son port reste confortable en période estivale. Par ailleurs, ce mode de réalisation de l'élément de maintien sous la forme d'une ceinture pectorale permet à l'utilisateur d'en ajuster facilement la position, notamment en hauteur, dans son dos. Ce qui n'est pas possible lorsque l'élément de maintien est réalisé sous la forme d'une chemise ou d'un T-shirt ou encore comprend des bretelles.

Selon une caractéristique de cette forme préférée de réalisation, le corps souple comprend une face interne destinée à être orientée vers le torse de l'utilisateur et, à l'opposé de la face interne, une face externe qui porte la coque élastiquement déformable. La coque s'étend alors en saillie du corps souple.

Selon une autre caractéristique de cette forme préférée de réalisation, les moyens de fermeture comprennent, au niveau d'une extrémité du corps souple, des crochets destinés à coopérer avec des boucles, également appelées astrakan ou velours, offertes par la face externe du corps souple. Selon cette caractéristique les moyens de fermeture sont formés par un système à boucles et crochets ou astrakan et crochets formant ainsi un système auto-agrippant du type « *velours-crochet* » tel que celui commercialisé sous la marque Velcro®. Cette manière de réaliser les moyens de fermeture permet de faciliter l'ajustement du corps souple.

Selon encore une autre caractéristique de cette forme préférée de réalisation, la face interne du corps souple comprend au moins une bande d'adhérence. De manière plus particulièrement préférée, la face interne du corps souple comprend au moins deux bandes d'adhérences disposées de part et d'autre d'une zone de fixation de la coque souple. La mise en oeuvre de bandes d'adhérence permet d'éviter un déplacement du corps souple sur le torse de l'utilisateur pendant son sommeil de sorte que le corps reste à sa position de placement initiale, de préférence dans la partie supérieure du dos bien au-dessus de la région lombaire, ce qui optimise l'efficacité de l'élément en volume pour empêcher l'utilisateur de dormir sur le dos.

Selon une variante de cette caractéristique chaque bande d'adhérence comprend au moins un bourrelet de silicone.

Selon l'invention la coque peut être fixée de toute manière appropriée sur le corps souple, pour autant que cette fixation s'opère au moins en partie, et de préférence en totalité, par l'intermédiaire du volet, et qu'elle soit permanente comme évoqué précédemment. Selon une variante préférentielle de l'invention, le rebord de fixation de la coque (*i.e.* le volet) est cousu sur le corps souple, par exemple selon une suite de points de couture qui suit un contour circulaire, le long de la périphérie du volet (qui présente lui-même avantageusement une forme circulaire), ce qui permet d'assurer une solidarisation irréversible de l'élément en volume et du corps souple (sauf à détruire le moyen de fixation, c'est-à-dire à découdre le volet et le corps souple).

Selon une caractéristique de la forme préférée de réalisation, le corps souple possède une longueur comprise entre 900 mm et 1300 mm. Une telle longueur du corps souple le rend utilisable pour des utilisateurs de différentes tailles ou corpulence.

Selon une autre caractéristique de cette forme préférée de réalisation, le corps souple possède une largeur comprise entre 60 mm et 120 mm.

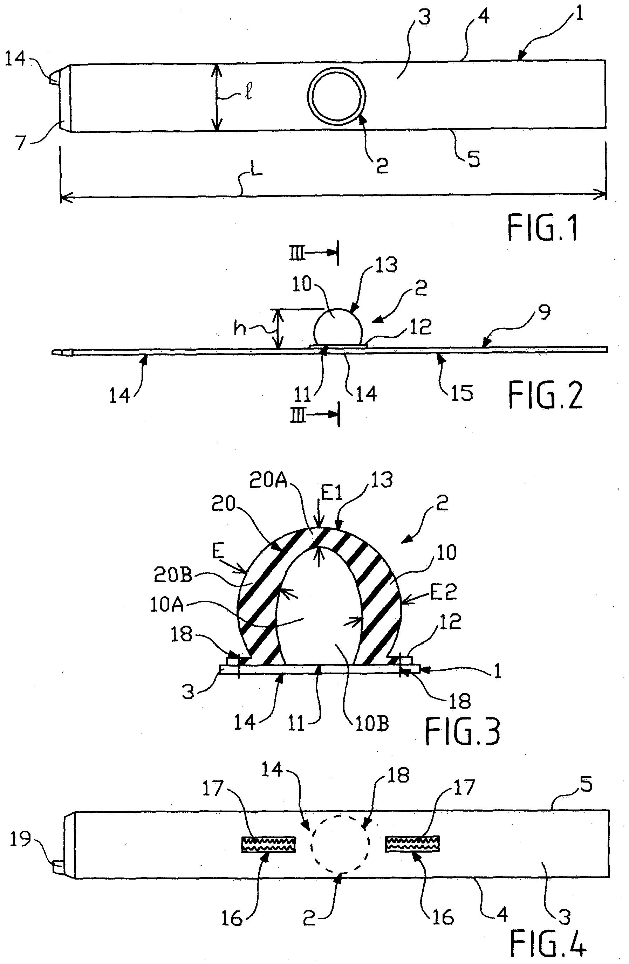

Avantageusement, comme illustré par la figure 3, la coque est formée par une paroi dont l'épaisseur varie spatialement, de façon à conférer à la coque des propriétés de déformation anisotropes. En d'autres termes, la coque présente dans ce mode de réalisation avantageux, de préférence du fait de son épaisseur variable, une aptitude à la déformation qui n'est pas uniforme, de sorte que certaines zones de la coque sont par exemple plus facilement déformable (en flexion notamment) que d'autres. Cette disposition technique permet d'obtenir un compromis remarquable entre efficacité et confort d'utilisation, en ajustant finement la déformabilité de la coque, de préférence afin que cette dernière se déforme plus facilement lorsqu'elle est soumise à une pression majoritairement axiale s'exerçant sur son sommet en direction de la face proximale. Ainsi, la coque se déformera plus difficilement tant que l'utilisateur dort sur le côté et vient éventuellement (par exemple dans une tentative inconsciente de rejoindre une position de sommeil dorsale) appuyer latéralement la coque contre le matelas de son lit. Cette rigidité latérale de la coque permet de dissuader de façon relativement efficace l'utilisateur de rejoindre une position de sommeil sur le dos. Il ne peut cependant pas être totalement exclu que l'utilisateur puisse rejoindre malgré tout une position dorsale, y compris de façon transitoire pour passer par exemple d'une position de sommeil sur le côté droit à une position de sommeil sur le côté gauche (ou l'inverse). Dans ce cas, lorsque l'utilisateur se trouve sur le dos, la partie de la coque (en l'occurrence son sommet) en appui contre le matelas se déformera facilement (du fait de son épaisseur moindre), ce qui conduira l'utilisateur à ressentir (sans le réveiller) une gêne suffisamment importante pour l'inciter à ne pas rester sur le dos, mais en aucun cas une douleur qui pourrait le réveiller. Afin d'obtenir cet effet technique particulièrement intéressant, la paroi formant la coque est avantageusement formée par une portion de paroi sommitale et une portion de paroi latérale qui s'élève entre la face proximale et ladite portion de paroi sommitale, la portion de paroi sommitale présentant une épaisseur qui est, au moins localement, sensiblement inférieure à l'épaisseur de ladite portion de paroi latérale, de façon que la portion de paroi sommitale se déforme plus facilement que la portion de paroi latérale, afin de réduire le niveau de douleur en position sur le dos sans nuire pour autant à l'efficacité du dispositif. Il n'est pas indifférent de noter que ce comportement mécanique spécifique et les caractéristiques correspondantes qui permettent de l'obtenir forme une invention indépendante (qui n'est pas ici revendiquée), qui peut être par exemple définie de la façon suivante : dispositif anti-ronflement comprenant un élément en volume ainsi qu'un élément de maintien dans le dos d'un utilisateur dudit élément en volume, caractérisé en ce que ledit élément en volume comprend au moins une paroi élastiquement déformable qui délimite ou contribue à délimiter un volume intérieur fermé ou ouvert, vide ou rempli au moins partiellement d'un matériau souple de remplissage, l'épaisseur de ladite paroi variant spatialement, de façon à conférer audit élément en volume des propriétés de déformation anisotropes. Bien entendu, cette invention indépendante ne nécessite pas forcément le recours à une coque, à un rebord (*a fortiori* en forme de volet), à un assemblage permanent de l'élément en volume et de l'élément de maintien, même si le recours à tout ou partie de ces caractéristiques serait préféré.

Les objets assignés à l'invention sont également atteints à l'aide d'un procédé de fabrication d'un dispositif anti-ronflement comprenant une étape dans laquelle on fournit ou on fabrique un élément en volume, ainsi qu'une étape dans laquelle on fournit ou on fabrique un élément de maintien dans le dos d'un utilisateur dudit élément en volume, ledit procédé étant caractérisé en ce que ledit élément en volume comprend au moins une coque élastiquement déformable et présentant une face proximale sensiblement plane ou concave, ledit élément en volume comprenant en outre au moins un rebord qui borde ladite face proximale et qui se présente sous la forme d'un volet s'étendant entre une extrémité intérieure attachée à ladite coque et une extrémité extérieure libre, ledit procédé comprenant par ailleurs une étape dans laquelle on assemble de façon permanente le volet et l'élément de maintien, avec la face proximale orientée vers l'élément de maintien. De préférence, le procédé de fabrication comprend une étape de fabrication de ladite coque et dudit rebord en une pièce d'un seul tenant, de sorte que ledit rebord vient de matière avec la coque. Par exemple, ladite étape de fabrication de ladite coque et dudit rebord en une pièce d'un seul tenant est effectuée par moulage d'un matériau élastomère. De préférence le volet et l'élément de maintien sont cousus ensemble de façon à être assemblés de façon permanente.

Bien entendu, les différentes variantes et formes de réalisation de l'invention peuvent être associées les unes avec les autres selon diverses combinaisons dans la mesure où elles ne sont pas incompatibles ou exclusives les unes des autres.

### DESCRIPTIF SOMMAIRE DES DESSINS

Par ailleurs, diverses autres caractéristiques de l'invention ressortent de la description annexée effectuée en référence aux dessins qui illustrent une forme non limitative de réalisation d'un dispositif anti-ronflement conforme à l'invention.
- La figure 1 est une élévation du dispositif anti-ronflement selon l'invention en vue à partir de sa face interne destinée à être orientée vers le torse de l'utilisateur.
- La figure 2 est une élévation du dispositif anti-ronflement selon l'invention en vue à partir de côté.
- La figure 3 est une coupe du dispositif anti-ronflement selon la ligne III-III de la figure 2.
- La figure 4 est une élévation du dispositif anti-ronflement selon l'invention en vue à partir de sa face externe.

### MEILLEURE MANIERE DE REALISER L'INVENTION

Un dispositif anti-ronflement selon l'invention, tel qu'illustré à la figure 1, comprend un élément de maintien 1 dans le dos d'un utilisateur d'un élément en volume 2 destiné à induire un inconfort chez l'utilisateur lorsqu'il est couché sur le dos, afin de l'inciter, sans toutefois le réveiller si possible, à quitter cette position dorsale.

Selon l'exemple illustré, l'élément de maintien 1 est constitué par une ceinture pectorale qui comprend un corps souple allongé 3 élastiquement déformable dans le sens de la longueur au moins. Dans le cas présent, le corps 3 est formé par une bande d'un tissu ou d'un tricot dont les lisières forment les bords longitudinaux 4 et 5 du corps 3. Ainsi, les bords longitudinaux 4 et 5 sont doux et n'irritent pas la peau de l'utilisateur lors du contact direct avec cette dernière. La bande constitutive du corps 3 est de préférence réalisée de manière à être plus extensible dans le sens de la longueur que dans le sens de la largeur.

Le corps souple mesure entre 900 mm et 1300 mm de long et, de manière préférée, il possède une longueur L, mesurée entre ses deux extrémités, de l'ordre de 1180 mm. Une telle longueur permet, compte tenu de l'élasticité du corps souple, le port du dispositif selon l'invention par des utilisateurs adultes de différentes tailles ou corpulences.

Afin de permettre sa fermeture en boucle autour du torse de l'utilisateur, le corps souple comprend au niveau de l'une au moins de ses extrémités, l'extrémité gauche à la figure 1, des moyens de fermeture 7. Dans le cas présent, les moyens de fermeture 7 comprennent des crochets, du type Velcro®, destinés à coopérer avec des boucles, non visibles aux figures, offertes par la face externe 9 du corps souple sur une partie au moins de sa région située entre l'extrémité opposée aux moyens de fermeture 7 et l'élément en volume 2.

Par ailleurs, le corps souple 3 possède une largeur l, mesurée entre ses deux bords longitudinaux 4 et 5 comprise entre 60 mm et 120 mm et, de préférence, de l'ordre de 80mm. Une telle largeur permet d'offrir une surface de contact avec le torse de l'utilisateur suffisamment importante pour ne pas induire de gêne lors de l'ajustement du dispositif à la taille de l'utilisateur.

Selon l'invention l'élément de maintien 1 comprend, au niveau de sa face externe 9, en tant qu'élément en volume 2, une coque 10 qui s'étend en saillie du corps souple 3. Comme le montrent les figures 2 et 3, la coque 10, qui présente une forme bulbaire, comprend une face proximale 11, orientée vers l'élément de maintien 1, et venant en appui contre ce dernier. La face proximale 1 est bordée par au moins un rebord de fixation 12 sur l'élément de maintien. Le rebord 12 se présente sous la forme d'un volet s'étendant entre une extrémité intérieure attachée à ladite coque et une extrémité extérieure libre. Selon l'exemple illustré, le rebord 12 s'étend sur toute la périphérie de la face proximale de la coque 10. Par ailleurs la face proximale 11, y compris le rebord 12, est plane ou concave de manière à ne pas incommoder le porteur du dispositif selon l'invention lorsqu'il est dans une position autre que couché sur le dos. En effet, cette forme plane ou concave permet à la face proximale 11 d'épouser la forme du dos de l'utilisateur contre lequel est plaqué l'élément de maintien 2 qui porte la coque 10.

La coque 10 possède en outre une face distale 13 orientée à l'opposé de l'élément de maintien 1, qui est convexe et qui, selon l'exemple illustré, possède, sensiblement, une forme de calotte sphérique. De manière préférée, la coque 10 délimite une cavité 10A, de préférence remplie d'air, qui s'ouvre sur l'extérieur par l'intermédiaire d'une ouverture 10B ménagée à travers ladite face proximale 11, ladite ouverture est plaquée contre l'élément de maintien 1 (cf. figure 3) de sorte que ce dernier obture l'ouverture. La face proximale 11 est ainsi ouverte de manière à permettre une circulation de l'air entre l'intérieur (cavité 10A) de la coque 10 et le milieu extérieur au travers du corps 3 dont le textile constitutif est préférentiellement perméable à l'air.

Selon cet exemple de réalisation et compte tenu de la forme de calotte sphérique de la coque et du rebord périphérique, l'élément en volume présente une section droite axiale en forme générale d'oméga.

Afin de toujours induire une gêne à l'utilisateur, même très légèrement déformée quand celui-ci est en position couché sur le dos, la coque 10 possède une hauteur h, mesurée entre l'élément de maintien 1 et le sommet de sa face distale 13, supérieure à 50 mm, de préférence supérieure à 60mm et, par exemple, comprise entre 60 mm et 80 mm, et de manière préférée de l'ordre de 60 mm.

La coque 10 est de préférence réalisée par moulage en polymère naturel et/ou synthétique. Ainsi, la coque 10 et le rebord de fixation 12 forment un ensemble monobloc. Afin d'éviter des risques de réaction allergique, le polymère utilisé est de préférence un silicone de grade alimentaire ou médical.

La coque 10 est fixée sur la face externe du corps 3 de toute manière appropriée. A cette fin, ledit dispositif comprend un moyen de fixation 18 assurant un assemblage permanent du volet et de l'élément de maintien 1. Selon l'exemple illustré, la coque 10 est cousue sur le corps souple 3 au niveau du volet souple, du genre jupe radiale, formant le rebord de fixation 12, lequel vient de matière avec la coque. Une telle couture permet de réaliser une fixation solide et pérenne qui n'altère pas la souplesse de la zone de fixation 14 du corps 3 qui porte la coque 10.

Comme illustré à la figure 3, la coque 10 est formée par une paroi 20 dont l'épaisseur E varie spatialement, de façon à conférer à ladite coque 10 des propriétés de déformation anisotropes. La paroi 20 est ici plus précisément formée par une portion de paroi sommitale 20A et une portion de paroi latérale 20B qui s'élève entre la face proximale 11 et ladite portion de paroi sommitale 20A. La portion de paroi sommitale 20A présente une épaisseur E1 qui est, au moins localement, sensiblement inférieure à l'épaisseur E2 de ladite portion de paroi latérale 20B, de façon que la portion de paroi sommitale 20A se déforme plus facilement que la portion de paroi latérale 20B. Ceci permet de rendre moins rigide le sommet de la coque 10, qui s'aplatira plus aisément pour éviter que l'utilisateur ne ressente une douleur trop importante lorsqu'il se retrouve sur le dos, douleur qui pourrait notamment le réveiller complètement, ce qui n'est pas le but recherché. Un tel dispositif anti-ronflement à paroi à épaisseur variable constitue en tant que tel une invention, indépendamment de la présence ou non des autres caractéristiques exposées ici.

Le dispositif anti-ronflement ainsi réalisé est mis en oeuvre de la manière suivante. Avant de se coucher, l'utilisateur place le corps souple 3 autour de son torse en le disposant au niveau de ses pectoraux pour un homme et en dessous des seins pour une femme, la coque 10 étant bien entendu située dans le dos de l'utilisateur sans être en contact avec sa peau. Afin de faciliter l'ajustement du corps souple 3, l'extrémité gauche comprend une languette 19 permettant d'exercer une traction sur ladite extrémité avant d'appliquer les crochets constitutifs des moyens de fermeture 7 contre la face externe 9 du corps 3.

Afin, d'éviter un déplacement du corps souple le long du torse de l'utilisateur, la face interne 15 du corps souple 3 comprend au moins une bande d'adhérence 16 et, selon l'exemple illustré, deux bandes d'adhérences 16 qui sont situés de part et d'autre de la zone de fixation de la coque 10. Chaque bande d'adhérence 16 comprend au moins un et, de préférence, deux bourrelets de silicone 17.

Selon l'exemple décrit précédemment, l'élément de maintien 1 est formé par une bande souple 3. Toutefois, l'élément de maintien 1 pourrait être réalisé de toute autre manière appropriée et peut, par exemple, être constitué par un vêtement comme un tee-shirt ou une veste de pyjama.

Par ailleurs, selon l'exemple décrit précédemment l'élément en volume comprend une coque dont le volume intérieur est en partie vide. Toutefois, l'intérieur de la coque pourrait être rempli. De même, l'élément en volume pourrait être formé par une structure monobloc pleine ou encore une structure pleine ayant plusieurs éléments constitutifs.

Bien entendu, diverses autres modifications peuvent être apportées à l'invention dans le cadre des revendications annexées.

### POSSIBILITE D'APPLICATION INDUSTRIELLE

L'invention trouve son application industrielle dans la conception, la fabrication et l'utilisation de dispositifs anti-ronflement.

## Revendications

1. Dispositif anti-ronflement comprenant un élément en volume (2) ainsi qu'un élément (1) de maintien dans le dos d'un utilisateur dudit élément en volume (2), ledit élément en volume (2) comprenant au moins un rebord (12), **caractérisé en ce que** ledit élément en volume (2) comprend au moins une coque (10) élastiquement déformable et présentant une face proximale (11) sensiblement plane ou concave orientée vers l'élément de maintien (1), ledit rebord (12) bordant ladite face proximale (11) et se présentant sous la forme d'un volet s'étendant entre une extrémité intérieure attachée à ladite coque (10) et une extrémité extérieure libre, ledit dispositif comprenant par ailleurs un moyen de fixation assurant un assemblage permanent du volet et de l'élément de maintien (1).

2. Dispositif anti-ronflement selon la revendication 1, **caractérisé en ce que** ladite coque (10) présente, à l'opposé de la face proximale (11), une face distale (13) qui possède une forme convexe.

3. Dispositif anti-ronflement selon la revendication 1 ou 2 **caractérisé en ce que** l'élément en volume (2) présente une section droite axiale en forme d'oméga.

4. Dispositif anti-ronflement selon l'une des revendications 1 à 3 **caractérisé en ce que** ladite coque (10) délimite une cavité qui s'ouvre sur l'extérieur par l'intermédiaire d'une ouverture ménagée à travers ladite face proximale (11), ladite ouverture étant obturée par l'élément (1) de maintien, ladite cavité étant de préférence majoritairement vide, et de préférence totalement vide.

5. Dispositif anti-ronflement selon l'une des revendications 1 à 4 **caractérisé en ce que** ledit volet vient de matière avec ladite coque (10).

6. Dispositif anti-ronflement selon l'une des revendications 1 à 5 **caractérisé en ce que** ledit volet s'étend sur toute la périphérie de la face proximale (11).

7. Dispositif anti-ronflement selon l'une des revendications 1 à 6 **caractérisé en ce que** ladite face proximale (11) présente sensiblement une forme annulaire.

8. Dispositif anti-ronflement selon l'une des revendications 1 à 7 **caractérisé en ce que** ledit moyen de fixation comprend au moins un point de couture de sorte que ledit volet est cousu audit élément (1) de maintien.

9. Dispositif anti-ronflement selon l'une des revendications 1 à 8, **caractérisé en ce que** la coque (10) est adaptée pour se déformer sous une charge supérieure ou égale à 200 N.

10. Dispositif anti-ronflement selon l'une des revendications 1 à 9 **caractérisé en ce que** la coque (10) est formée par une paroi dont l'épaisseur varie spatialement, de façon à conférer à ladite coque des propriétés de déformation anisotropes, ladite paroi étant formée par une portion de paroi sommitale et une portion de paroi latérale qui s'élève entre la face proximale (11) et ladite portion de paroi sommitale, la portion de paroi sommitale présentant une épaisseur qui est, au moins localement, sensiblement inférieure à l'épaisseur de ladite portion de paroi latérale, de façon que la portion de paroi sommitale se déforme plus facilement que la portion de paroi latérale.

11. Dispositif anti-ronflement selon l'une des revendications 1 à 10, **caractérisé en ce que** l'élément de maintien (1) est constitué par une ceinture pectorale qui comprend un corps (3) souple allongé élastiquement déformable dans le sens de la longueur et pourvu au niveau d'une extrémité au moins, de moyens (7) de fermeture de la ceinture en boucle autour du torse de l'utilisateur, le corps souple (3) comprenant une face interne destinée à être orientée vers le torse de l'utilisateur et, à l'opposé de la face interne, une face externe qui porte la coque (10) élastiquement déformable.

12. Dispositif anti-ronflement selon la revendication 11, **caractérisé en ce que** la face interne du corps souple (3) comprend au moins une bande d'adhérence (16), et de préférence au moins deux bandes d'adhérences (16) disposées de part et d'autre d'une zone de fixation de la coque (10), chaque bande d'adhérence (16) comprenant de préférence au moins un bourrelet de silicone (17).

13. Procédé de fabrication d'un dispositif anti-ronflement comprenant une étape dans laquelle on fournit ou on fabrique un élément en volume (2), ainsi qu'une étape dans laquelle on fournit ou on fabrique un élément (1) de maintien dans le dos d'un utilisateur dudit élément en volume (2), ledit procédé étant **caractérisé en ce que** ledit élément en volume (2) comprend au moins une coque (10) élastiquement déformable et présentant une face proximale (11) sensiblement plane ou concave, ledit élément en volume (2) comprenant en outre au moins un rebord (12) qui borde ladite face proximale (11) et qui se présente sous la forme d'un volet s'étendant entre une extrémité intérieure attachée à ladite coque (10) et une extrémité extérieure libre, ledit procédé comprenant par ailleurs une étape dans laquelle on assemble de façon permanente le volet et l'élément de maintien (1), avec la face proximale (11) orientée vers l'élément de maintien (1).

14. Procédé de fabrication selon la revendication 13 **caractérisé en ce qu'**il comprend une étape de fabrication de ladite coque (10) et dudit rebord (12) en une pièce d'un seul tenant, par exemple par moulage d'un matériau élastomère, de sorte que ledit rebord (12) vient de matière avec la coque (10).

15. Procédé de fabrication selon la revendication 13 ou 14 **caractérisé en ce que** le volet et l'élément de maintien (1) sont cousus ensemble de façon à être assemblés de façon permanente.

## Patentansprüche

1. Antischnarchvorrichtung, umfassend ein Volumselement (2) sowie ein Halteelement (1) im Rücken eines Benutzers des Volumselements (2), wobei das Volumselement (2) mindestens einen Rand (12) umfasst, **dadurch gekennzeichnet, dass** das Volumenselement (2) mindestens eine Hülle (10) umfasst, welche elastisch verformbar ist und eine proximale Fläche (11) aufweist, die im Wesentlichen eben oder konkav ist und zu dem Halteelement (1) gerichtet ist, wobei der Rand (12) die proximale Fläche (11) begrenzt und sich in der Form eines Flügels präsentiert, der sich zwischen einem inneren Ende, das an der Hülle (10) angebracht ist, und einem freien äußeren Ende erstreckt, wobei die Vorrichtung außerdem ein Befestigungsmittel umfasst, das eine permanente Fixierung des Flügels und des Halteelements (1) gewährleistet.

2. Antischnarchvorrichtung nach Anspruch 1, **dadurch gekennzeichnet, dass** die Hülle (10), gegenüber der proximalen Fläche (11), eine distale Fläche (13) aufweist, die eine konvexe Form hat.

3. Antischnarchvorrichtung nach Anspruch 1 oder 2, **dadurch gekennzeichnet, dass** das Volumselement (2) einen axialen geraden Querschnitt in Omega-Form aufweist.

4. Antischnarchvorrichtung nach einem der Ansprüche 1 bis 3, **dadurch gekennzeichnet, dass** die Hülle (10) einen Hohlraum begrenzt, der sich zur Außenseite mittels einer Öffnung öffnet, die quer über die proximale Fläche (11) angeordnet ist, wobei die Öffnung durch das Halteelement (1) verschlossen wird, wobei der Hohlraum vorzugsweise mehrheitlich leer ist und vorzugsweise gänzlich leer ist.

5. Antischnarchvorrichtung nach einem der Ansprüche 1 bis 4, **dadurch gekennzeichnet, dass** der Flügel mit der Hülle (10) einteilig ist.

6. Antischnarchvorrichtung nach einem der Ansprüche 1 bis 5, **dadurch gekennzeichnet, dass** sich der Flügel über den gesamten Umfang der proximalen Fläche (11) erstreckt.

7. Antischnarchvornchtung nach einem der Ansprüche 1 bis 6, **dadurch gekennzeichnet, dass** die proximale Fläche (11) im Wesentlichen eine Ringform aufweist.

8. Antischnarchvorrichtung nach einem der Ansprüche 1 bis 7, **dadurch gekennzeichnet, dass** das Befestigungsmittel mindestens eine Nahtstelle aufweist, so dass der Flügel an das Halteelement (1) angenäht ist.

9. Antischnarchvorrichtung nach einem der Ansprüche 1 bis 8, **dadurch gekennzeichnet, dass** die Hülle (10) geeignet ist, sich unter einer Kraft von größer oder gleich 200 N zu verformen.

10. Antischnarchvorrichtung nach einem der Ansprüche 1 bis 9, **dadurch gekennzeichnet, dass** die Hülle (10) durch eine Wand gebildet ist, deren Dicke räumlich variiert, um der Hülle anisotrope Verformungseigenschaften zu verleihen, wobei die Wand durch einen Scheitelwandabschnitt und einen lateralen Wandabschnitt gebildet wird, der sich zwischen der proximalen Fläche (11) und dem Scheitelwandabschnitt erhebt, wobei der Scheitelwandabschnitt eine Dicke aufweist, die, mindestens lokal, wesentlich kleiner ist als die Dicke des lateralen Wandabschnitts, so dass sich der Scheitelwandabschnitt leichter verformt als der laterale Wandabschnitt.

11. Antischnarchvorrichtung nach einem der Ansprüche 1 bis 10, **dadurch gekennzeichnet, dass** das Halteelement (1) aus einem Brustgurt besteht, welcher einen länglichen, nachgiebigen Körper (3) umfasst, der in der Längsrichtung elastisch verformbar ist und, mindestens auf der Höhe eines Endes, mit Verschlussmitteln (7) des Schnallengurts um die Brust des Benutzers versehen ist, wobei der nachgiebige Körper (3) eine Innenfläche, die dazu bestimmt ist, zur Brust des Benutzers gerichtet zu werden, und, gegenüber der Innenfläche, eine Außenfläche, welche die elastisch verformbare Hülle (10) trägt, aufweist.

12. Antischnarchvorrichtung nach Anspruch 11, **dadurch gekennzeichnet, dass** die Innenfläche des nachgiebigen Körpers (3) mindestens ein Haftband (16) und vorzugsweise mindestens zwei Haftbänder (16) umfasst, die auf beiden Seiten einer Befestigungszone der Hülle (10) angeordnet sind, wobei jedes Haftband (16) vorzugsweise mindestens einen Silikonwulst (17) umfasst.

13. Verfahren zur Herstellung einer Antischnarchvorrichtung, umfassend einen Schritt, in dem ein Volumselement (2) bereitgestellt oder hergestellt wird, sowie einen Schritt, in dem ein Haltelement (1) im Rücken eines Benutzers des Volumenselements (2) bereitgestellt oder hergestellt wird, wobei das Verfahren **dadurch gekennzeichnet ist, dass** das Volumselement (2) mindestens eine Hülle (10) umfasst, welche elastisch verformbar ist und eine proximale Fläche (11) aufweist, die im Wesentlichen eben oder konkav ist, wobei das Volumselement (2) außerdem mindestens einen Rand (12) umfasst, welcher die proximale Fläche (11) begrenzt und welcher sich in der Form eines Flügels präsentiert, der sich zwischen einem inneren Ende, das an der Hülle (10) angebracht ist, und einem freien äußeren Ende erstreckt, wobei das Verfahren außerdem einen Schritt umfasst, in dem auf permanente Weise der Flügel und das Halteelement (1) fixiert werden, wobei die proximale Fläche (11) zu dem Halteelement (1) gerichtet ist.

14. Herstellungsverfahren nach Anspruch 13, **dadurch gekennzeichnet, dass** dieses einen Schritt der Herstellung der Hülle (10) und des Rands (12) in einem Stück aus einem Teil umfasst, beispielsweise durch Formen eines elastomeren Materials, so dass der Rand (12) mit der Hülle (10) einteilig ist.

15. Herstellungsverfahren nach Anspruch 13 oder 14, **dadurch gekennzeichnet, dass** der Flügel und das Halteelement (1) zusammengenäht sind, um auf permanente Weise fixiert zu sein.

## Claims

1. Anti-snoring device, comprising a bulky element (2) and an element (1) for holding said bulky element (2) in the back of a user, said bulky element (2) comprising at least one rim (12), **characterised in that** said bulky element (2) comprises at least one elastically deformable shell (10) having a substantially planar or concave proximal face (11) oriented towards the holding element (1), said rim (12) bordering said proximal face (11) and being in the form of a flap extending between an inner end attached to said shell (10) and a free outer end, said device moreover comprising a fixing means providing permanent assembly of the flap and holding element (1).

2. Anti-snoring device according to claim 1, **characterised in that** said shell (10) has, opposite to the proximal face (11), a distal face (13) that has a convex shape.

3. Anti-snoring device according to claim 1 or claim 2, **characterised in that** the bulky element (2) has an axial cross section in the shape of an omega.

4. Anti-snoring device according to one of claims 1 to 3, **characterised in that** said shell (10) delimits a cavity that opens onto the outside through an opening provided through said proximal face (11), said opening being closed off by the holding element (1), said cavity preferably being mainly empty, and preferably completely empty.

5. Anti-snoring device according to one of claims 1 to 4, **characterised in that** said flap is made in one piece with said shell (10).

6. Anti-snoring device according to one of claims 1 to 5, **characterised in that** said flap extends over the entire periphery of the proximal face (11).

7. Anti-snoring device according to one of claims 1 to 6, **characterised in that** said proximal face (11) has substantially an annular shape.

8. Anti-snoring device according to one of claims 1 to 7, **characterised in that** said fixing means comprises at least one stitching point so that said flap is stitched to said holding element (1).

9. Anti-snoring device according to one of claims 1 to 8, **characterised in that** the shell (10) is suitable for deforming under a load greater than or equal to 200 N.

10. Anti-snoring device according to one of claims 1 to 9, **characterised in that** the shell (10) is formed by a wall, the thickness of which varies spatially, so as to confer anisotropic deformation properties on said shell, said wall being formed by a top wall portion and a lateral wall portion that rises between the proximal face (11) and said top wall portion, the top wall portion having a thickness that is, at least locally, substantially less than the thickness of said lateral portion, so that the top wall portion deforms more easily than the lateral wall portion.

11. Anti-snoring device according to one of claims 1 to 10, **characterised in that** the holding element (1) is formed by a pectoral belt that comprises an elongate flexible body (3) that is elastically deformable in the direction of the length and is provided, at at least one end, with means (7) for closing the belt in a loop around the torso of the user, the flexible body (3) comprising an internal face intended to be oriented towards the torso of the user and, opposite to the internal face, an external face that carries the elastically deformable shell (10).

12. Anti-snoring device according to claim 11, **characterised in that** the internal face of the flexible body (3) comprises at least one adhesion band (16), and preferably at least two adhesion bands (16) disposed on either side of a zone for fixing the shell (10), each adhesion band (16) preferably comprising at least one silicone bead (17).

13. Method for manufacturing an anti-snoring device, comprising a step in which a bulky element (2) is supplied or manufactured, as well as a step in which an element (1) for holding said bulky element (2) in the back of a user is supplied or manufactured, said method being **characterised in that** said bulky element (2) comprises at least one elastically deformable shell (10) having a substantially planar or concave proximal face (11), said bulky element (2) further comprising at least one rim (12) that borders said proximal face (11) and is in the form of a flap extending between an inner end attached to said shell (10) and a free outer end, said method moreover comprising a step in which the flap and the holding element (1) are permanently assembled, with the proximal face (11) oriented towards the holding element (1).

14. Manufacturing method according to claim 13, **characterised in that** it comprises a step of manufacturing said shell (10) and said rim (12) in a single piece, for example by moulding an elastomer material, so that said rim (12) is made in one piece with the shell (10).

15. Manufacturing method according to claim 13 or claim 14, **characterised in that** the flap and the holding element (1) are stitched together so as to be assembled permanently.
